# EUROPEAN PATENT APPLICATION

(11) **EP 4 471 154 A1**
(43) Date of publication of application: **04.12.2024**
(21) Application number: 23746138.9
(22) Date of filing: 17.01.2023
(51) Int. Cl.: C12Q 1/42, C12Q 1/68, C07K 16/00, C12N 15/09, G01N 33/15, A61K 38/08

(54) **USE OF KININ OR DERIVATIVE THEREOF IN TREATING VCI, PSCI, OR CSVD**

(30) Priority: 30.01.2022 CN 202210113865
(71) Applicant: Zonhon Biopharma Institute, Inc., Changzhou, Jiangsu 213125 (CN)
(72) Inventor: MA, Bruce Yong, hangzhou, Jiangsu (CN); JIANG, Chenyang, hangzhou, Jiangsu 213125 (CN); ZHUANG, Yu, hangzhou, Jiangsu 213125 (CN); XU, Zhen, hangzhou, Jiangsu 213125 (CN); XU, Dan, hangzhou, Jiangsu 213125 (CN); WANG, Jun, hangzhou, Jiangsu 213125 (CN)
(74) Representative: Axe PI
(86) International application number: PCT/CN2023/072640
(87) International publication number: WO 2023/143256

(57) **Abstract**

Provided is use of kinin or derivative thereof, in particular long-acting kinin or derivative thereof, in treating vascular cognitive impairment (VCI), post-stroke cognitive impairment (PSCI), or cerebral small vessel disease (CSVD). VCI and PSCI are generally associated with cognitive function impairment and psychoactive behavioral symptom. Drugs for treating cognitive impairment in VCI and PSCI include cholinesterase inhibitors, non-competitive N-methyl-D-asparagine receptor antagonists, and drugs for treating psychoactive behavioral symptom include antidepressants. Patients with chronic CSVD may have symptoms such as cognitive disorder, movement disorder, emotional disorder, defecation disorder and other symptoms. Cholinesterase inhibitors and non-competitive N-methyl-D-asparagine receptor antagonists can be used for cognitive disorder, and antidepressants are generally used for depression. It is found that the kinin or the derivative thereof, in particular the long-acting kinin or the derivative thereof, can be used for treating VCI, PSCI, CSVD, or the like, thus providing new method for treating VCI, PSCI, or CSVD.

## Description

### TECHNICAL FIELD

The present invention relates to kinins or derivatives thereof, in particular, the use of long-acting kinins or its derivatives in the treatment of vascular cognitive impairment (VCI), post-stroke cognitive impairment (PSCI), or cerebral small vessel disease (CSVD).

### BACKGROUND OF THE INVENTION

With the development of society and aging population, the incidence of cerebrovascular disease is increasing year by year, which seriously affects the quality of life of patients and their families. The concept of vascular cognitive impairment (VCI) was first proposed by Hachinski and Bowler in 1993. It refers to a large group of syndromes ranging from mild cognitive impairment to dementia caused by risk factors of cerebrovascular disease (such as hypertension, diabetes, hyperlipidemia, etc.), obvious cerebrovascular disease (such as cerebral infarction and cerebral hemorrhage, etc.), or less obvious cerebrovascular disease (such as leukoaraiosis, chronic cerebral ischemia). (Chinese Guidelines for the Diagnosis and Treatment of Vascular Cognitive Impairment (2019*)*, *"*Chinese Guidelines for the Diagnosis and Treatment of Vascular Cognitive Impairment " in Chinese Guidelines for the Prevention and Treatment of Stroke (2021*)* )

Patients with vascular cognitive impairment have cognitive impairment and psychoactive behavioral symptoms. The cognitive impairment of some patients mainly presents with the impairment of abstract thinking, concept formation and transformation, thinking flexibility, information processing speed and other functions, while memory is relatively preserved. Some patients present with multi-domain impairment and memory impairment. Drugs for cognitive impairment in VCI include cholinesterase inhibitors and non-competitive N-methyl-D-asparagine receptor antagonists. Psychoactive behavioral symptoms include abnormal perception, thinking, mood, and behavior, such as apathy, depression, irritability, agitation/aggression, and drugs for the treatment include antidepressant drugs such as serotonin reuptake inhibitors (*"Chinese* Guidelines for the Diagnosis and Treatment of Vascular Cognitive Impairment "). Post-stroke cognitive impairment (PSCI), cognitive impairment caused by cerebral small vessel disease (CSVD) and Alzheimer's disease (AD) with vascular lesions are important subtypes of VCI.

Post-stroke cognitive impairment emphasizes that stroke triggers cognitive dysfunction, which also involves cognitive impairment and psychoactive behavioral symptoms. Considering that there is some overlap in neuropathological and neurobiochemical mechanisms among PSCI, VCI and AD, the Chinese Expert Consensus on the Management of Post-Stroke Cognitive Impairment (2021 *)* recommends to refer to relevant research and evidence of VCI and AD. The drugs for the treatment of cognitive impairment generally include acetylcholinesterase inhibitors and non-competitive N-methyl-D-asparagine receptor antagonists (memantine). The drugs for the treatment of post-stroke depression generally include antidepressants. The latest "*2021ESO*/*EAN Joint Guidelines on Post-stroke Cognitive Impairment"* issued by the European Stroke Organization points out that there is no drug for the treatment of post-stroke cognitive impairment. Only one trial specifically focused on post-stroke cognitive impairment and had no positive results. The guideline also points out that there is a significant shortage of high-quality data from randomized controlled trials (RCTS) for PSCI.

Cerebral small vessel disease (CSVD) is a series of clinical, imaging and pathological syndromes caused by a variety of risk factors affecting small arteries, arterioles, capillaries, venules and venules in the brain. It is divided into acute CSVD and chronic CSVD. Acute CSVD can lead to ischemic stroke. At present, it is recommended to refer to the prevention and treatment of acute ischemic stroke, such as antihypertensive, thrombolysis, antiplatelet, and lipid-lowering therapy. Patients with chronic CSVD may have cognitive impairment, movement disorder, emotional disorder, defecation disorder and other symptoms. It is generally recommended that symptomatic treatment be carried out after a clear diagnosis. For cognitive impairment caused by CSVD, cholinesterase inhibitors and non-competitive N-methyl-D-asparagine receptor antagonists can be selected, and antidepressants such as serotonin reuptake inhibitors are generally selected for depression (" *Chinese Expert Consensus on the Diagnosis and Treatment of Cerebral Small Vessel Disease* ").

### SUMMARY OF THE INVENTION

The first technical problem addressed by this application is to provide a new drug for the treatment of vascular cognitive impairment. In particular, the use of kinin or its derivatives in the preparation of drugs for the treatment or improvement of vascular cognitive impairment is provided.

The invention also provides a pharmaceutical composition, comprising kinin or a derivative thereof for the treatment or improvement of vascular cognitive impairment.

The present invention also provides a method of treating or improving vascular cognitive impairment by administering kinin or a derivative thereof to the patient.

Vascular cognitive impairment includes but is not limited to post-stroke cognitive impairment, cognitive impairment caused by cerebral small vessel disease, Alzheimer's disease with vascular lesion, etc.

Preferably, the vascular cognitive impairment is post-stroke cognitive impairment.

Preferably, the vascular cognitive impairment is cognitive impairment due to cerebral small vessel disease.

Preferably, the vascular cognitive impairment is Alzheimer's disease with vascular lesion.

The second technical problem addressed by this application is to provide a new drug for the treatment of cerebral small vessel disease. In particular, the use of kinin or its derivatives in the preparation of drugs for the treatment or improvement of cerebral small vessel disease is provided.

The present application also provides a pharmaceutical composition, comprising kinin or a derivative thereof for the treatment or improvement of cerebral small vessel disease.

The present application also provides a method of treating or improving cerebral small vessel disease by administering kinin or a derivative thereof to the patient.

The above kinin is bradykinin or lysyl bradykinin.

The above kinin derivative is long-acting kinin.

Peptide drugs generally have the problems of poor stability and short half-life in vivo, especially for the treatment of chronic diseases, which often require long-term and frequent administration. The degradation of lysyl bradykinin/bradykinin in vivo is mainly involved in aminopeptidase, carboxypeptidase and angiotensin ACEII. The dynamic balance of bradykinin in vivo is achieved based on kallikrein and degrading enzyme system. Lysyl bradykinin and bradykinin are more unstable than common peptides, with reported half-life of only seconds, and exogenously administered kinins are quickly inactivated by kallikretin hydrolysis in vivo.

In order to improve the stability of peptide drugs, prolong the half-life in vivo, and make them long-acting, there are two main methods: 1. Molecular modification of peptides usually using amino acid substitution or cyclization, PEG modification, fusion of long-acting fragments (fusion with Fc fragment, fusion with human serum albumin), and conjugation with fatty acids. 2. Pharmaceutics to make long-acting polypeptide drugs.

In specific embodiments of the present application, two methods are used to prolong the half-life of the kinin, that is PEG modification and molecular modification.

About PEG modification, in specific embodiments of the application, the modifiers adopted include SPA, SCM, and SS modifiers; it can be straight-chain or branched PEG modifier.

Preferably, the PEG modifier is straight-chain PEG modifier.

Preferably, the PEG modifier molecular weight is 2KD-20KD.

About molecular modification, in specific embodiments of the present application, cysteine (Cys) is introduced at any position in the wild-type lysyl bradykinin or bradykinin sequence. Preferably, the first Lys of wild-type lysyl bradykinin is mutated to Cys, which is CRPPGFSPFR.

In the specific embodiment of the present application, any number and any kind of amino acids are inserted between the first and second amino acid residues at the N-terminus of wild-type lysyl-bradykinin, namely KXRPPGFSPFR, X represents any number and any kind of amino acids. Any kind of amino acid was inserted before the first amino acid residue at the N-terminus of wild-type bradykinin, namely XRPPGFSPFR, where X represents any number and any kind of amino acids. The results showed that the activity was better after the N-terminus prolongation. Preferably, one or two or three amino acids are inserted. Preferably, one or two arginines (Arg) are inserted.

In specific embodiments of the present application, derivative of lysyl bradykinin or bradykinin consists of two or more lysyl bradykinin monomers in series(e.g. m14), or consists of two or more bradykinin monomers in series, or consists of lysyl bradykinin monomers and bradykinin monomers in series (e.g. m12). The lysyl bradykinin or bradykinin monomer may be wild-type lysyl bradykinin or bradykinin, or variant after substitution, insertion, or deletion of some amino acids in the wild-type lysyl bradykinin or bradykinin.

In a specific embodiment of the present application, Phe at posion 6 of wild-type lysyl bradykinin or Phe at posion 5 of wild-type bradykinin is mutated to other amino acids, such as the unnatural amino acids Igl(α-2-indolyl glycine), Thi(β-2-thiophenylalanine). LBK or BK mutated at the above sites is not easily recognized by enzymes in vivo, thus effectively extending its half-life in vivo.

Preferably, sequence of derivative of bradykinin or lysyl bradykinin is shown in SEQ ID NO: 14.

PEG modification and molecular modification used in specific embodiments of the present application are only used to illustrate that the half-life of kinin can be extended by the above means, but not to limit the method of extending half-life of kinin, and those skilled in the art may also use other polyethylene glycol modification and other molecular modification methods. Alternatively, other modification methods (as described above, fusion with Fc fragments, fusion with human serum albumin, conjugated fatty acids, pharmaceutics) may be used to modify the kinin to make it long-acting.

The kinins or derivatives thereof may be administered alone or in combination with other drugs, such as cholinesterase inhibitors, noncompetitive N-methyl-D-asparagine receptor antagonists, etc.

Administration methods include, but are not limited to, injection and oral administration. The injection methods include but are not limited to intravenous injection, subcutaneous injection, intramuscular injection, etc.

Well-recognized animal models of vascular cognitive impairment include: The 4-vessel occlusion (4-VO) method, modified 4-VO method, 3-stage 4-VO method, 2-vessel occlusion (2-VO) method, modified 2-VO, and modified common carotid artery occlusion (mCCAO) in rats , and bilateral CCA stenosis (BCAS) and asymmetric CCA surgery (ACAS) in mice *(Advances in Animal Models of Vascular Cognitive Impairment).*

The recognized animal models of cerebral small vessel disease mainly include bilateral common carotid artery ligation model, bilateral common carotid artery stenosis model (BCAS), and stroke-susceptible spontaneously hypertensive rat model, etc. ("*Chinese Expert Consensus on Translational Medical Research of Cerebral Small Vessel Disease* ").

As a more recognized experimental animal model in the study of vascular cognitive impairment and cerebral small vessel disease, the cerebral blood flow of BCAS model animals is significantly reduced, which causes chronic hypoperfusion injury, resulting in increased permeability of the blood-brain barrier and white matter damage, which in turn causes memory dysfunction and motor function impairment. The results of the present study showed that kallikrein I or its derivatives could significantly improve the impaired motor coordination function, impaired forelimb muscle strength, working memory, spatial learning and memory function in BCAS mice. Therefore, kallikrein I or its derivatives can be used to treat vascular cognitive impairment and cerebral small vessel disease.

In addition, post-stroke cognitive impairment, cognitive impairment caused by cerebral small vessel disease, and Alzheimer's disease with vascular lesions are important subtypes of vascular cognitive impairment. In the clinical treatment of these diseases, the same symptomatic treatment drugs are used. For cognitive impairment, the treatment guidelines or expert consensus for vascular cognitive impairment, post-stroke cognitive impairment, and cerebral small vessel disease generally recommend acetylcholinesterase inhibitors and non-competitive N-methyl-D-asparagine receptor antagonists (memantine). For psychoactive behavioral symptoms such as depression, antidepressants are generally recommended for the medical treatment. Therefore, kallikrein I or its derivatives can be used to treat various subtypes of vascular cognitive impairment, such as post-stroke cognitive impairment, cognitive impairment caused by cerebral small vessel disease, Alzheimer's disease with vascular lesions, etc.

The Kinin-kallikrein system (KKS) is involved in a variety of physiological and pathological processes, such as the regulation of cardiovascular, renal and nervous system functions. The KKS system includes kallikrein, kininogen, kinin, kinin receptors (B 1 and B2 receptors) and kininase. Kallikrein is a serine protease. It can be divided into two categories: plasma kallikrein I(PK) and tissue kallikrein I(TK), which play important physiological roles. At present, it is believed that human tissue kallikrein is composed of at least 15 members (KLK1-KLK15), among which there are many studies on tissue kallikrein I(KLK1). KLK1 plays a series of biological roles by converting kininogens into kinins and acting on corresponding receptors. In China, two kinds of kallikrein I for injection have been marketed, and the indications include microcirculation disorders and mild to moderate acute ischemic stroke.

Vascular cognitive impairment and cerebral small vessel disease are quite different from the approved indications of kallikrein I. In terms of pathological mechanism, acute ischemic stroke is brain tissue ischemia, hypoxic necrosis due to local cerebral blood flow reduction or blood supply interruption caused by stenosis, occlusion or thrombosis of the main arteries or branch arteries of the cerebral on the basis of vascular wall lesions caused by various reasons. VCI refers to a large group of syndromes ranging from mild cognitive impairment to dementia caused by cerebrovascular risk factors (such as hypertension, diabetes, hyperlipidemia, etc.), obvious cerebrovascular diseases (such as cerebral infarction and cerebral hemorrhage, etc.) or less obvious cerebrovascular diseases (such as leukoaraiosis, chronic cerebral ischemia, etc.). The abnormal function of NVU (neurovascular unit) plays an important role in the pathogenesis of cerebral small vessel disease. Changes in the structure or function of NVU caused by any reasons can lead to CSVD. The common mechanisms include chronic cerebral ischemia and hypoperfusion, endothelial dysfunction, blood-brain barrier disruption, interstitial fluid reflux disorder, inflammatory response and genetic factors, etc. There is an interaction between different mechanisms. (*The Neurovaseular Unit Coming of Age: a Journey Through Neurovaseular Coupling in Health and Disease)*

In addition, the diagnosis and treatment of acute ischemic stroke, vascular cognitive impairment and cerebral small vessel disease are quite different. The most effective treatment for acute ischemic stroke is vascular recanalization therapy within the time window, including intravenous thrombolysis, mechanical thrombectomy, angioplasty, etc. The success rate of treatment is closely related to disease course. Drug therapy includes antiplatelet, anticoagulation, defibrination, volume expansion, vasodilatation, statins, neuroprotective drugs, etc. Cognitive impairment and psychoactive behavioral symptoms are common in VCI and PSCI. Drugs for cognitive impairment in VCI and PSCI include cholinesterase inhibitors, non-competitive N-methyl-D-asparagine receptor antagonists. Drugs for psychoactive behavioral symptoms are as antidepressant drugs such as serotonin reuptake inhibitors. Patients with chronic cerebral small vessel disease may have symptoms such as cognitive impairment, movement disorders, emotional disorders, and defecation disorders. It is generally recommended that symptomatic treatment be carried out after a definite diagnosis. For cognitive impairment caused by CSVD, cholinesterase inhibitors and non-competitive N-methyl-D-asparagine receptor antagonists can be selected. For depression, antidepressants such as 5-hydroxytryptamine reuptake inhibitors are generally selected.

The application found that kinin or its derivatives can be used to treat vascular cognitive impairment, post-stroke cognitive impairment, cerebral small vessel disease, etc, which provides a new method for the treatment of vascular cognitive impairment, post-stroke cognitive impairment and cerebral small vessel disease.

### BRIEF DESCRIPTION OF THE DRAWINGS

FIG. 1: Lys-BK and its variants activated phosphorylation of downstream ERK1/2 and CREB, where β-actin was the internal reference. p-represents the phosphorylation of the corresponding protein; control was buffer; Lys-BK-M06-EH was the mixture of m06 and serum after incubation
FIG. 2: Affinity assay of PEG conjugates of Lys-BK and its variant to B2 receptor (reporter gene assay)
FIG. 3: PEG conjugates of Lys-BK and its variant activated phosphorylation of downstream ERK1/2 and CREB, where β-actin was the internal reference. p-represents the phosphorylation of the corresponding protein; control was buffer
FIG. 4: Effect of drugs on latency to fall in rotarod test (Day 13).
FIG. 5: Effect of drugs on latency to fall in rotarod test (Day 26).
FIG. 6: Effect of drugs on latency to fall in wire hang test (Day 20).
FIG. 7: Effect of drugs on latency to fall in wire hang test (Day 28).
FIG. 8: Effect of drugs on alternation behavior in Y Maze test (Day 29).
FIG. 9: Effect of drugs on alternation behavior in Y Maze test (Day 44).
FIG. 10: Effect of drugs on platform latency in Morris maze test (Day 48).
FIG. 11: Effect of drugs on sucrose preference in SPT (Day 57).

### DETAILED DESCRIPTION OF THE INVENTION

Unless otherwise specified, the technical terms or abbreviations of this application have the following meanings:
Lys-BK: wild-type lysyl bradykinin, with sequence identical to native human lysyl bradykinin; The amino acid sequence was KRPPGFSPFR.

BK: wild-type bradykinin, with sequence identical to native human bradykinin; The amino acid sequence was RPPGFSPFR.

Mutant: variant obtained by substitution, insertion, or deletion of some amino acids in wild-type lysyl bradykinin or bradykinin.

Derivatives: including not only the various mutants mentioned in the embodiments of this application, but also further modified peptides, fusion proteins (including but not limited to albumin fusion, Fc fusion, etc.) and various forms of modifications on the basis of Lys-BK/BK mutant in this application.

Polyethylene glycol: PEG, usually formed by the polymerization of ethylene oxide, has branched, linear, and multi-arm forms. Ordinary polyethylene glycol has hydroxyl group at each end. If one end is blocked with methyl group, methoxy polyethylene glycol (mPEG) is obtained.

Polyethylene glycol modifier: PEG modifier refers to PEG derivatives with functional groups, which are activated polyethylene glycol and can be used for protein and peptide drug modification. The polyethylene glycol modifier used in this application was purchased from ZonHonBiopharma Institute , Inc or Jenkem Technology Co., LTD. The actual molecular weight of PEG modifier can be 90%-110% of the labeled value. For example, molecular weight of PEG5K can be 4.5-5.5 kDa.

M-SPA-5K: straight-chain monomethoxy polyethylene glycol succinimidyl propionate with molecular weight of about 5kDa, the structure is shown in general formula (1), n is an integer from 98 to 120,

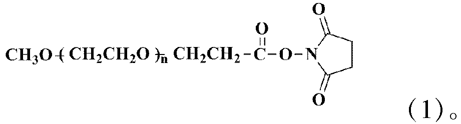

M-SPA-2K: straight-chain monomethoxy polyethylene glycol succinimidyl propionate with molecular weight of about 2kDa, the structure is shown in general formula (1), and n is an integer from 37 to 45.

M-SPA-10K: straight-chain monomethoxy polyethylene glycol succinimidyl propionate with molecular weight of about 10 kDa, the structure is shown in general formula (1), and n is an integer from 200 to 245.

M-SPA-20K: straight-chain monomethoxy polyethylene glycol succinimidyl propionate with molecular weight of about 20kDa and structure as shown in general formula (1), and n is an integer from 405 to 495.

M-SCM-5K: straight-chain monomethoxy polyethylene glycol succinimidyl acetate with molecular weight of about 5kDa, the structure is shown in general formula (2), and n is an integer from 99 to 120.

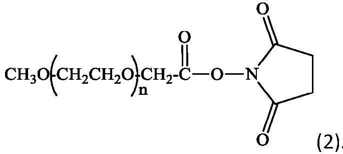

M-SS-5K: straight-chain monomethoxy polyethylene glycol succinimidyl succinate with molecular weight of about 5kDa, the structure is shown in general formula (3), and n is an integer from 98 to 119.

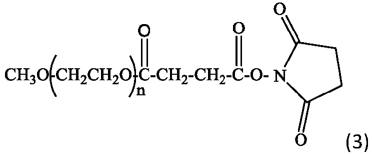

M-YNHS-10K: branched-chain monomethoxy polyethylene glycol succinimidyl acetate with molecular weight of about 10kDa, the structure is shown in general formula (4), n is an integer from 98 to 120,

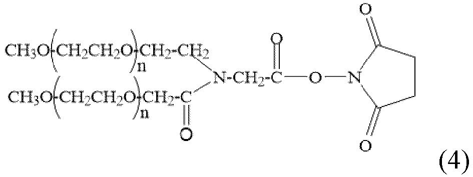

### Example 1 Design, synthesis of lysyl bradykinin/bradykinin variants

Based on the sequence of lysyl bradykinin/bradykinin and the possible degradation pathways in vivo, a series of variants were designed (Table 1-1), mainly by replacing natural amino acids with structurally similar natural amino acids and unnatural amino acids (m01~m04 and m07, among which m02 is a BK variant). In order to identify the minimum active unit for biological activity/function, partially truncated or extended peptide variants (m05, m06, M08-M11) were designed; Cysteines were introduced (m13, m15, m16) to further improve the antioxidant properties of the variant. To further improve the stability of peptides, lysyl bradykinin/bradykinin monomer was connected in series (m12 was consisted of Lys-BK and BK, m14 was consisted of two Lys-BK). Based on the m13 peptide, m15 and m16 variants were designed to compare the effect of N-terminal extension on drug efficacy. The purity of all peptides was > 95%.

**Table 1-1 Sequences of lysyl bradykinin/bradykinin variants**

| Peptide | Sequences | SEQ ID NO |
|---|---|---|
| Lys-BK | KRPPGFSPFR | 1 |
| Lys-BKm01 | KRPPG{THI}SPFR | 2 |
| Lys-BKm02 | RPPG{THI}SPFR | 3 |
| Lys-BKm03 | KRPPGHSPFR | 4 |
| Lys-BKm04 | KRPPGFSPHR | 5 |
| Lys-BKm05 | KRPPG{THI}SPF | 6 |
| Lys-BKm06 | KRPPG{THI}SPFRR | 7 |
| Lys-BKm07 | KRPPG{THI}SP{THI}R | 8 |
| Lys-BKm08 | PG{THI}SPFR | 9 |
| Lys-BKm09 | {THI}SPFR | 10 |
| Lys-BKm10 | SPFR | 11 |
| Lys-BKm11 | KRRPPG{THI}SPFR | 12 |
| Lys-BKm12 | KRPPG{THI}SPFRRPPG{THI}SPFR | 13 |
| Lys-BKm13 | CRRPPG{THI}SPFR | 14 |
| Lys-BKm14 | KRPPG{THI}SPFRKRPPG{THI}SPFR | 15 |
| Lys-BKm15 | CRPPG{THI}SPFR | 16 |
| Lys-BKm16 | CRRRPPG{THI}SPFR | 17 |

### Example 2 Stability studies of lysyl bradykinin/bradykinin and its variants

The stability of lysyl bradykinin/bradykinin was assessed by evaluating its stability in serum system in vitro. 2mg/ml peptide and SD rat serum were mixed at volume ratio of 85: 15, incubated at 37°C for 30min, 60min, 90min, 120min, 180min, etc, terminated by adding 3% trichloroacetic acid (50% volume of the above peptide serum mixture system) and effectively removing matrix interference in the serum.

Further monitor the degradation of peptide samples in the serum system to evaluate its stability basing on UPLC-RP-UV₂₁₄ₙₘ method. RP analysis method: Protein BEH C4 2.1 × 150mm as chromatographic column, 1.7 µm, column temperature 30°C, mobile phase A: 0.1% TFA/H₂O, mobile phase B: 0.1% TFA/ACN, gradient: 0-5min, 5%B; 5-10min, 5-40% B; 10-30min, 40-60%B; 30-32min, 60-100% B; 32 to 35min, 5% B.

**Table 2-1 Stability of lysyl bradykinin and its variants in serum system**

| Peptide | Residual time in serum system |
|---|---|
| Lys-BK | 30~60min |
| Lys-BKm01 | 60~90min |
| Lys-BKm02 | 60~90min |
| Lys-BKm03 | 60~90min |
| Lys-BKm04 | >180min |
| Lys-BKm05 | >180min |
| Lys-BKm06 | 60^{~}90min |
| Lys-BKm07 | 60^{~}90min |
| Lys-BKm11 | 60^{~}90min |
| Lys-BKm12 | 90-120min |
| Lys-BKm13 | 60^{~}90min |
| Lys-BKm14 | 90-120min |
| Lys-BKm15 | 60^{~}90min |
| Lys-BKm16 | 60^{~}90min |

The results in Table 2-1 showed that the designed polypeptide variants had better stability in serum than the wild type lysyl bradykinin, which also indicated that they had certain advantages in stability and drug efficacy in vivo.

### Example 3 Evaluation of in vitro activity of lysyl bradykinin/bradykinin and its variants

### I. Affinity activity to B2 receptor based on reporter gene assay

The pharmacological action of lysyl bradykinin/bradykinin in vivo is achieved by binding to B2 receptors and activating downstream signaling pathways

The cell line B2-NFAT-CHO-K1 stably transfected with B2 receptor reporter gene was constructed based on CHO-K1 host cells and the cell library was constructed. The B2-NFAT-CHO-K1 cell library was used to evaluate the binding activity of lysyl bradykinin/bradykinin or its variants or PEG modified products to B2 receptor in vitro.

Detection methods: The cells were cultured in 96-well plates at 5 × 10⁴ cells/well, 37°C, 5% CO₂ for 16-20 hours, and then gradient diluted standard and test solutions were added. After 5 hours, added Bio-Lite luciferase detection reagent and the chemiluminescence intensity was measured. GraphPad was used to make four-parameter equation curve and calculate the relative activity according to the EC50 value. Table 3-1 listed the relative affinity activity of the peptide variants to B2 receptor, among which Lys-BKm11, Lys-BKm13, Lys-BKm15 and Lys-BKm16 had higher activity than the wild type Lys-BK.

**Table 3-1 Relative affinity activity of Lys-BK and its variants to B2 receptor**

| Peptide | Relative affinity activity | Peptide | Relative affinity activity |
|---|---|---|---|
| Lys-BK | 100% | Lys-BKm08 | <10% |
| Lys-BKm01 | 100% | Lys-BKm09 | 0% |
| Lys-BKm02 | 100% | Lys-BKm10 | 0% |
| Lys-BKm03 | <10% | Lys-BKm11 | 110% |
| Lys-BKm04 | <10% | Lys-BKm12 | 100% |
| Lys-BKm05 | <10% | Lys-BKm13 | 115% |
| Lys-BKm06 | 90% | Lys-BKm14 | 100% |
| Lys-BKm07 | <10% | Lys-BKm15 | 125% |
| | | Lys-BKm16 | 120% |

### II. Activity analysis based on key target proteins in downstream signaling pathways

B2-NFAT-CHO-K1, which was CHO-K1 cells overexpressing B2 receptor, was in vitro intervened with Lys-BK/BK or its different variants. And the changes of p-CREB and p-ERK1/2 (P means phosphorylation, serine or threonine on the protein is phosphorylated and activated, and produces a series of effects in the pathway), which was B2 receptor downstream signals, were detected by WB method to compare the activity differences between the test substances. Extracellular regulated protein kinases 1/2 (ERK1/2), cAMP-response element binding protein (CRBE) are direct signal molecules after B2R activation. After stroke, the activation of CREB and ERK1/2 can prevent the inflammatory response and neuronal damage after ischemia, so the activation level of CREB and ERK1/2 can reflect B2R activating ability of the test substance. At the same time, the significance of the neuroprotective effect is reflected.

CHO-K1(B2R+) cells in logarithmic growth phase were collected and adjusted to 3× 10⁶ cells/mL with medium and seeded in cell culture plates at 3 × 10⁵ cells/ well. The cells were cultured in a CO₂ incubator at 37.0°C and 5.0% CO₂ for 24-48 hours. When the cell density was more than 90%, the cells were washed with PBS, and 1ml 1µM Lys-BK/BK or its variant sample was added to each well, while the control group was F12 medium. The cell culture plates were incubated at 37.0°C, 5.0% CO₂ for 5min, and the cell culture plates were washed with PBS. Protein lysate (RIPA, PMSF, phosphatase inhibitor) was added, 80µL/ well, and the bottom of the well was ground back and back with a scraping rod to accelerate cell lysis. The cell debris and lysate was transferred to a centrifuge tube and centrifuged at 12000rpm,
4 °C for 10min. The supernatant was aspirated, mixed with 5×Loading Buffer at volume ratio of 4:1, and boiled in metal bath at 100°C for 10min. The same amount of protein samples were loaded into 10% SDS-PAGE for gel running, membrane transfer, blocking, antibody incubation, and luminescence identification.

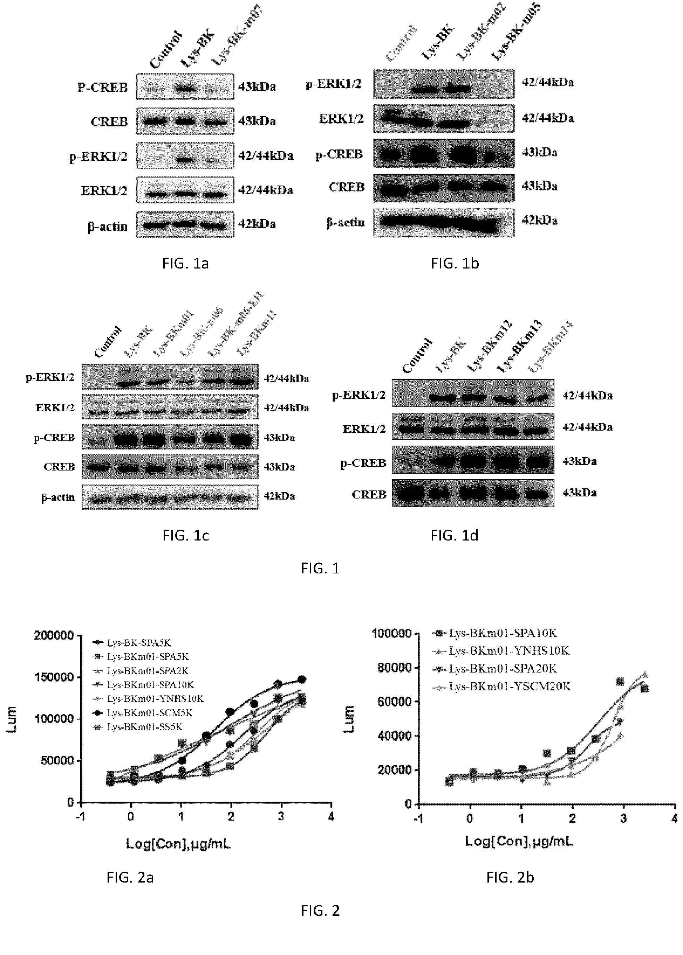

As shown in FIG. 1, Lys-BK/BK or its variants can activate the phosphorylation of downstream ERK1/2 and CREB proteins, which was consistent with the action mechanism of this drug. The in vitro biological activity of the samples was qualitatively evaluated by judging the phosphorylation levels of the downstream ERK1/2 and CREB proteins. The evaluation results showed that Lys-BKm05 and Lys-BKm07 lost the activity of activating downstream pathways, while LBKm01, m02, m06, m11, m12, m13, m14, m15 and m16 all showed the activity of activating downstream pathways.

The above two in vitro activity evaluation results showed that lysyl bradykinin/bradykinin after C-terminal truncation lost B2R affinity and lost activity of activating downstream pathway. Lysyl bradykinin missing N-terminal lysine did not affect B2R affinity and activity of activating downstream pathway. Lysyl bradykinin missing other N-terminal amino acids lost B2R affinity and lost activity of activating downstream pathway. The minimum active unit was RPPGFSPFR. Both N-terminal lengthening (m11) or introduction of cysteine (m13) can increase B2R affinity activity. Figure 1d showed that the phosphorylation of CREB protein in m12, 13, and 14 groups was significantly higher than that in LBK group, revealing that m12, 13, and 14 could better activate the downstream pathways and show better in vitro activity than LBK.

### Example 4 Preparation of PEG conjugates of lysyl bradykinin/bradykinin and its variants

PEG modification of lysyl bradykinin/bradykinin and its variants was carried out by conventional methods in the art, specific to particular variants and PEG modifiers, with slight changes in some parameters, which can be obtained by those skilled in the art through a limited number of experiments, and there are no technical obstacles. Taking M-SPA-5K modified wild-type BK as an example, the preparation process is as follows:

### I. Preparation of PEG conjugates

Peptide samples were dissolved with pH7.0 sodium dihydrogen phosphate/disodium hydrogen phosphate buffer so that the final concentration was 10mg/mL. PEG was added to the peptide solution, and the mass ratio of peptide to M-SPA-5K was 1:7, and the mixture was stirred slowly until it was evenly mixed. The reaction was carried out at 2-8°C for 1h.

### II. Purification of the reaction mixture

In the first purification step, the chromatographic conditions were as follows:
GE Superdex75 medium was used as the purification filler, and the purification mobile phase was pH7.0-7.5, 20mM sodium dihydrogen phosphate/disodium hydrogen phosphate buffer containing 0.2M NaCl.

Sample loading: After the reaction, the above reaction mixture was directly loaded.

Elution: after sample loading, the chromatographic column was washed with mobile phase of no less than 1.5 column volume, and eluted samples were collected step by step according to the trend of UV₂₁₄ₙₘ.

In the second purification step, the chromatographic conditions were as follows:
C18 reversed-phase column achieved separation based on the polarity difference, the product purification yield can reach more than 80%, and the free PEG can be completely removed.

### III. Purity analysis of PEG conjugate samples

### (1)HPLC purity analysis

The detection was performed by high performance liquid chromatography (HPLC) according to the General rule 0512 of Chinese Pharmacopoeia (2020 Edition). The chromatographic type was SEC(size-exclusion chromatogrphy chromatography), the mobile phase was 20mM PB7.0+ 10% IPA, the chromatographic column was TSK G2000, and the collection condition was 214nm.

RP purity analysis conditions were as follows: Protein BEH C4 2.1 × 150mm, 1.7µm as chromatographic column, mobile phase A was 0.1% TFA/H₂O, and mobile phase B was 0.1% TFA/ACN.

The purity results showed that the PEG modified lysyl-bradykinin variants had uniform peaks, no obvious impurity peaks, and the purity was > 95%.

### (2) Purity analysis by SDS-PAGE

The sample purity was determined by SDS-polyacrylamide gel electrophoresis method, the fifth method of electrophoresis method in Chinese Pharmacopoeia 0541 version 2020, and 12.5% SDS-PAGE was used for sample detection.

The results of electrophoresis showed that the prepared PEG modified lysyl-bradykinin variants had uniform bands, no heterozygous bands were observed, and the purity was > 95%.

In addition, lysyl bradykinin/bradykinin or its variants only have a theoretically modification site at the N-terminus, so the PEG modifications were all N-terminal single-modified.

### Example 5 In vitro evaluation of PEG conjugates of lysyl bradykinin/bradykinin and its variants

### I. Stability

The stability of PEG conjugates of lysyl bradykinin and its variants was indirectly evaluated by evaluating their stability in serum system in vitro, as determined in Example 2. PEG conjugates of lysyl bradykinin and its variants had residual time of more than 6h in the serum system in vitro (Table 5-1), indicating significant increase in stability. The stability of the branched chain conjugate Lys-BKm01-YNHS 10K was better than that of the straight chain conjugate Lys-BKm01-SPA10K at the same molecular weight of PEG.

**Table 5-1 Stability of PEG conjugates of lysyl bradykinin and its variants in the serum system**

| Peptides | Residual time in serum system |
|---|---|
| Lys-BK-SPA5K | >6h |
| Lys-BKm01-SPA2K | >6h |
| Lys-BKm01-SPA5K | >6h |
| Lys-BKm01-SPA10K | >6h |
| Lys-BKm01-YNHS10K | >12h |
| Lys-BKm01-SCM5K | >6h |
| Lys-BKm01-SS5K | >6h |

### II. In vitro activity

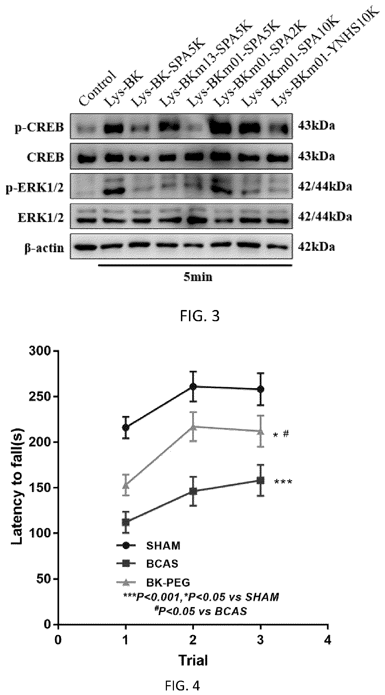

In vitro B2-receptor affinity activity and activity of activating downstream signaling pathway of PEG conjugates of lysyl bradykinin and its variants were tested, as in Example 3. Lysyl bradykinin or its variants after PEG modification still had affinity to B2 receptor (Figure 2a, 2b. And still can activate the phosphorylation of key proteins,which were downstream signaling pathways of B2 receptors (Figure 3). Conjugates using PEG modifiers of different structural types and molecular weights, including 2K to 20K, straight chain and branch, and different structures (SPA, SCM, SS, etc.) still retained the binding activity to B2 receptor. The results showed that the activity of straight-chain PEG conjugates was higher than that of branched-chain PEG conjugates at the same molecular weight, and this result was true for both 10K and 20K PEG. As shown in Figure 2a, the EC₅₀ value of Lys-BKm01-SPA10K and Lys-BKm01-YNHS 10K were 90.8µg/ml and 346.6µg/ml, respectively, indicating that the linear PEG conjugate was nearly 4-fold more active than the branched PEG conjugate. Among 2K, 5K, 10K and 20K PEG conjugates with the same PEG structure type, the activity of 2K and 10K PEG conjugates were relatively higher.

The activity results of activating the downstream signaling pathway of PEG conjugates of lysyl bradykinin and its variants (FIG. 3) showed that PEG conjugates of the peptides retained the activity of activating the phosphorylation of ERK1/2 and CREB, which was downstream of the B2 receptor. Although the activity decreased compared with that of Lys-BK, especially ERK1/2 phosphorylation. With the same SPA5K-PEG modifier, Lys-BKm13-SPA5K had the highest activity among different peptide variants. With the same peptide variant Lys-BKm01, 2K and 10K PEG conjugates showed the highest activity, and the linear PEG conjugate Lys-BKM01-SPA10K showed higher activity than the branched PEG conjugate Lys-BKM01-YNHS10K.

In conclusion, PEG conjugates of lysyl bradykinin and its variants were more stable, and still retained the B2 receptor affinity and retained the activity to activate downstream signaling pathway.

### Example 6 Comparison of drug effects of BK-PEG (BK was wild-type lysyl-bradykinin and PEG was SPA5K) in mouse BCAS model

BK-PEG (5mg/kg, BK was wild-type lysyl bradykinin, PEG was SPA5K) was injected intravenously in mouse model of bilateral common carotid artery stenosis (BCAS) with cerebral small vessel ischemia injury. The protective effects of repeated administration of BK-PEG on cerebral small vessel ischemia injury were observed and compared.

### I. Grouping and experimental design

Mouse model of bilateral common carotid artery stenosis (BCAS) with cerebral small vessel ischemic injury was established by spring constriction of bilateral common carotid artery.

Preparation of BCAS cerebral small vessel injury model: The BCAS cerebral small vessel ischemic injury model was used to achieve persistent chronic forebrain ischemia in mice. The mice were anesthetized with isoflurane. First, the mice were anesthetized in the induction box of the anesthesia machine, and then the mice were placed supine and connected with a breathing mask. The skin was prepared and sterilized, the neck was cut in the middle, and the bilateral common carotid artery was separated. The bilateral common carotid arteries were constricted with custom-made spring (diameter: 0.08mm, inner diameter: 0.18mm, pitch: about 0.5mm, total length: 2.5mm) to block most of the blood flow, resulting in the decrease of cerebral blood supply and persistent chronic cerebral ischemia. The neck skin was sutured, sterilized, and animals were returned to the cage for feeding.

The experiment was divided into three groups: SHAM operation group (SHAM group), model control group (BCAS group) and BK-PEG group.

The animals were given drugs on the third day after BCAS surgery. The BK-PEG group was treated with BK-PEG (5mg/kg) via the tail vein twice a week for 7 weeks, while the BCAS and SHAM groups were treated with the same volume of normal saline via the tail vein twice a week.

Rotarod test was performed to detect the motor coordination function of mice on the 13th and 26th day after surgery. The mice were placed on the rotarod (30mm diameter ×60mm length) in the opposite direction of rotation. Uniformly accelerated the rotarod at 20rpm/min to 40rpm/min and then maintained. The time of mice on the rotarod (that is, the latency to fall) was recorded. Each mouse was tested 3 to 5 times, with an interval of 20 to 30 minutes. The mean value was taken as the latency to fall, and longer latency to fall indicated better motor coordination.

Wire hang test was performed to detect the damage of forelimb muscle strength on the 20th and 28th days after surgery. The forelimbs of the mice were suspended on a steel wire (1.5mm diameter×60cm length), and the time of the mice on the wire (that is, the latency to fall) was recorded. Each mouse was tested 3 to 5 times, with an interval of 20 to 30min each time, and the mean value was taken as the latency to fall. Longer latency to fall indicated less muscle damage.

Y Maze test was performed to detect the impairment of working memory of mice on the 29th and 44th day after surgery. Y maze was a "Y-shaped" experimental device composed of three identical arms 120°away from each other. Each arm was 30cm long, 8cm wide and 15cm high. The test taked advantage of the nature of rodents to explore new environments and was used for researches such as learning and memory. The mice were placed in the Y maze and allowed to move freely. The order of the mice entering each arm with all four paws was recorded for 7min. All arm entries were defined as the number of arm entries, and the number of consecutive entries into all three arms was defined as the number of alternation, with the alternation score as the Y Maze evaluation index. Alternation score = [number of alternation/(number of arm entries -2)]* 100. Animals with less than eight arm enteres during the experiment were eliminated (because the data did not reflect precise changes). Higher alternation scores indicated less impaired working memory.

Morris Maze Test was performed on D48 to test the spatial learning and memory ability of the mice. The water temperature of the maze was maintained at (23 ± 2) °C , and the pool was divided into four quadrants (E, S, W, N) with four entry points marked on the pool wall. A camera with a display system was placed above the maze to synchronously record the movement trajectory of the mice. The reference objects outside the maze were kept constant during the training period to aid mice positioning. Taking the platform latency (the time from entering the water to finding the platform) as an indicator, the shorter platform latency mean the milder impairment of spatial learning and memory.

The sucrose preference test (SPT) was performed to detect the depressive behavior of mice on the 57th day after surgery. The sucrose drinking test was a measure of the absence of anhedonia. The absence of anhedonia was lack of interest in rewarding stimuli that was sign of affective disorder, including depression. Depression was assessed by the intake of 1% sucrose water and drinking water. The percentage of sucrose water intake in the total amount of sucrose water and drinking water was used to reflect the depression index. The higher score of sucrose preference mean the weaker degree of depression.

### II. Results

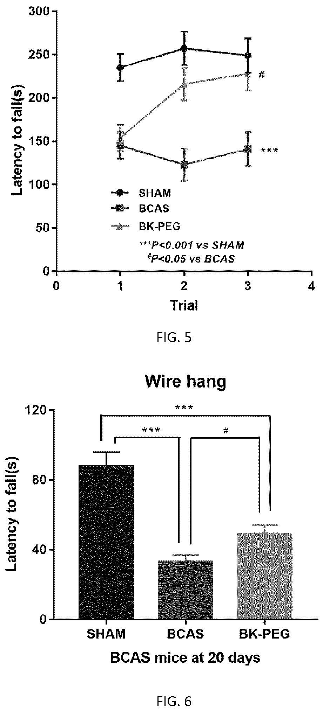

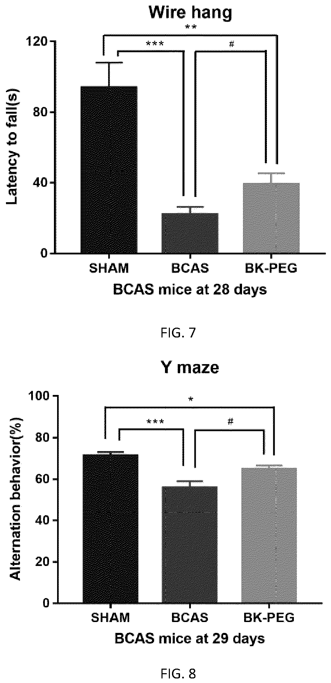

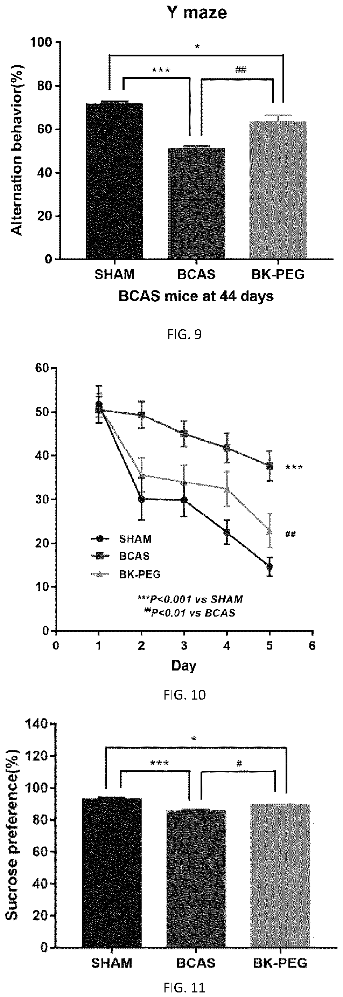

Compared with SHAM group, BCAS group showed significant statistical differences in the results of rotarod test, wire hang test, Y-maze test, Morris maze test and sucrose preference test, indicating that BCAS modeling led to the impairment of motor coordination, forelimb muscle strength, working memory and spatial learning and memory, and induced depression behavior in animals. Compared with BCAS model group, BK-PEG can significantly improve the motor coordination, forelimb muscle strength, working memory and spatial learning and memory impairment caused by BCAS model, and reduce BCAS-induced depression behavior. BCAS model is a more recognized experimental animal model in the study of vascular cognitive impairment and cerebral small vessel disease, so the test drugs can be used to treat vascular cognitive impairment, post-stroke cognitive impairment, and cerebral small vessel disease. The results were shown in Figures 4-11 and Tables 6-1 to 6-5.

**Table 6-1 Effect of intravenous administration on latency to fall(s) in rotarod test**

| Number of observations | Groups | Number of animals | Latency to fall(s) on 13d (mean value ± standard error) | Latency to fall(s) on 26d (mean value ± standard error) |
|---|---|---|---|---|
| 1 | SHAM | 16 | 216±11.8 | 235±15.7 |
| | BCAS | 17 | 112±11.5 | 145±15.2 |
| | BK-PEG | 17 | 153±115 | 154±15.2 |
| 2 | SHAM | 16 | 261±16.4 | 257±19.2 |
| | BCAS | 17 | 146±16.0 | 123±18.6 |
| | BK-PEG | 17 | 217±16.0 | 216±18.6 |
| 3 | SHAM | 16 | 258±17.6 | 249±19.9 |
| | BCAS | 17 | 158±17.1 | 141±19.3 |
| | BK-PEG | 17 | 212±17.1 | 228±19.3 |

**Table 6-2 Effect of intravenous administration on latency to fall(s) in wire hang test**

| Groups | Number of animals | Latency to fall(s) on 20d (mean value ± standard error) | Latency to fall(s) on 28d (mean value ± standard error) |
|---|---|---|---|
| SHAM | 16 | 88±8.0 | 94±14.1 |
| BCAS | 17 | 33±3.8*** | 22±4.4*** |
| BK-PEG | 17 | 49±5.3***^{#} | 39±6.4**^{#} |

| | | | |
|---|---|---|---|
| ***P<0.001, **P<0.01, compared with SHAM group; #P<0.05, compared with BCAS group. | | | |

**Table 6-3 Effect of intravenous administration on alternation behavior in Y Maze test**

| | Groups | Number of animals | Alternation behavior (%) in Y Maze (mean value±standard error) |
|---|---|---|---|
| 29days | SHAM | 16 | 71.3±1.7 |
| | BCAS | 17 | 55.8±3.2*** |
| | BK-PEG | 17 | 64.8±1.8*^{#} |
| 44days | SHAM | 16 | 71.2±1.6 |
| | BCAS | 17 | 50.5±1.8*** |
| | BK-PEG | 17 | 63.2±3.2*^{##} |

| | | | |
|---|---|---|---|
| ***P<0.001, **P<0.01, *P<0.05, compared with SHAM group; ##P<0.01, #P<0.05, compared with BCAS group. | | | |

**Table 6-4 Effect of intravenous administration on platform latency (s) in Morris maze test**

| Day | Groups | Number of animals | Platform Latency (s) (mean value ± standard error) |
|---|---|---|---|
| 1 | SHAM | 12 | 51.7±4.29 |
| | BCAS | 16 | 50.5±2.98 |
| | BK-PEG | 15 | 51.5±2.71 |
| 2 | SHAM | 12 | 30.1±4.82 |
| | BCAS | 16 | 49.3±3.07 |
| | BK-PEG | 15 | 35.6±3.94 |
| 3 | SHAM | 12 | 29.9±3.79 |
| | BCAS | 16 | 45.0±2.88 |
| | BK-PEG | 15 | 34.0±3.86 |
| 4 | SHAM | 12 | 22.5±2.71 |
| | BCAS | 16 | 41.8±3.36 |
| | BK-PEG | 15 | 32.4±3.97 |
| 5 | SHAM | 12 | 14.7±2.14 |
| | BCAS | 16 | 37.7±3.44 |
| | BK-PEG | 15 | 22.9±3.88 |

**Table 6-5 Effect of intravenous administration on sucrose water preference in SPT**

| Groups | Number of animals | Sucrose preference on 57d (%) (mean value±standard error) |
|---|---|---|
| SHAM | 14 | 92.5±1.60 |
| BCAS | 17 | 85.0±1.28*** |
| BK-PEG | 17 | 88.9±0.88*^{#} |

| | | |
|---|---|---|
| ***P<0.001, *P<0.05, compared with SHAM group; #P<0.05, compared with BCAS group. | | |

### Example 7 Comparison of drug effects of BK-PEG (BK was m11 and PEG was SPA5K) in mouse BCAS model

BK-PEG 5mg/kg (BK was m11 and PEG was SPA5K) was given intravenously for multiple times in mouse model of bilateral common carotid artery stenosis (BCAS) with cerebral small vessel ischemic injury. The protective effects of repeated administration on cerebral small vessel ischemic injury were observed and compared.

### I. Grouping and experimental design

Mouse model of bilateral common carotid artery stenosis (BCAS) with cerebral small vessel ischemic injury was established by spring constriction of bilateral common carotid artery. The BCAS small vessel injury model was prepared as in Example 6.

There were two groups in the experiment: model group (BCAS group) and BK-PEG(5mg/kg) group.

The animals were given drugs on the 7th day after BCAS surgery. The BK-PEG group was given BK-PEG via the tail vein twice a week for 6 weeks, while the BCAS group was given the same volume of normal saline via the tail vein twice a week.

Rotarod test was used to detect the motor coordination function of mice in the 6th week. Morris maze test was used to detect the impairment of spatial learning and memory of mice in the 1st week after the end of administration.

### II. Results

Compared with BCAS group, BK-PEG group showed a tendency to improve the results of rotarod test and a significant difference in the results of Morris maze test, indicating that BK-PEG could improve the impairment of motor coordination and spatial learning and memory induced by BCAS. The BCAS model is a more recognized experimental animal model in the study of vascular cognitive and cerebral small vessel disease, so the test drugs can be used to treat vascular cognitive impairment, post-stroke cognitive impairment, and cerebral small vessel disease. Details of the results were shown in Tables 10-11.

**Table 10 Effect of intravenous administration on latency to fall in rotarod test (s, Mean±SEM)**

| Groups | Latency to fall(s) in the 6th week |
|---|---|
| BCAS | 57.96±7.56 |
| BK-PEG | 70.00±9.20 |

**Table 11 Effect of intravenous administration on total distance (mm, Mean±SEM) and platform latency (s, Mean±SEM) in Morris maze**

| Groups | Total distance (mm) | Platform latency (s) |
|---|---|---|
| BCAS | 19347.84±2230.90 | 148.14±1.86 |
| BK-PEG | 13800.48±1106.96* | 132.10±9.87 |

| | | |
|---|---|---|
| * : indicates P < 0.05 compared with the model group | | |

### Example 8 Comparison of drug effects of BK-PEG (BK was m13 and PEG was SPA2K) in mouse BCAS model

BK-PEG 15mg/kg (BK was m13, PEG was SPA2K) was given subcutaneously for multiple times in mouse model of bilateral common carotid artery stenosis (BCAS) with cerebral small vessel ischemic injury. The protective effects of repeated BK-PEG on cerebral small vessel ischemic injury were observed and compared.

### I. Grouping and experimental design

Mouse model of bilateral common carotid artery stenosis (BCAS) with cerebral small vessel ischemic injury was established by spring constriction of bilateral common carotid artery.

The BCAS small vessel injury model was prepared as in Example 6.

There were two groups in the experiment: model group (BCAS group) and BK-PEG (15mg/kg) group.

The animals were given drugs on the 7th day after BCAS surgery. The BK-PEG group was injected subcutaneously with BK-PEG via the tail vein twice a week for 8 weeks.The model group (BCAS group) was injected subcutaneously with the same volume of saline via the tail vein twice a week.

The Rotarod test was used to detect the motor coordination function of mice in the 8th week after administration. Morris maze test was used to detect the impairment of spatial learning and memory of mice in the 1st week after administration.

### II. Results

Compared with the model group (BCAS group), the BK-PEG group showed a trend of improvement in rotarod test and a statistically significant difference in the results of the maze test, indicating that BK-PEG could significantly improve the impairment of motor coordination, spatial learning and memory in BCAS animals. BCAS model is a more recognized experimental animal model in the study of vascular cognitive impairment and cerebral small vessel disease, so the test drugs can be used to treat vascular cognitive impairment, post-stroke cognitive impairment, and cerebral small vessel disease. The results were shown in Tables 12 and 13.

**Table 12 Effect of subcutaneous injection on latency to fall in rotarod test (s, Mean±SEM)**

| Groups | Latency to fall (s, Mean±SEM) |
|---|---|
| BCAS | 47.51±6.15 |
| BK-PEG | 65.26±12.14 |

**Table 13 Effect of subcutaneous injection on total distance (mm, Mean±SEM) and platform latency (s, Mean±SEM) in Morris maze**

| Groups | Total distance (mm) | Platform latency (s) |
|---|---|---|
| BCAS | 10755.83±1180.36 | 64.74±6.92 |
| BK-PEG | 6659.77±1212.91* | 37.00±6.17* |

| | | |
|---|---|---|
| * : indicates P < 0.05 compared with the model group | | |

BCAS model was performed in examples 6 to 8, which was a more recognized experimental animal model in the study of vascular cognitive impairment and cerebral small vessel disease. All the test drugs retained the activity of receptor affinity and activating the downstream signaling pathway. All the drugs could improve the impaired motor coordination function, forelimb muscle strength, working memory, spatial learning and memory function, and depression behavior in BCAS model mice.

Except for the test drugs in examples 6-8, similar effects were observed when BCAS model mice were given other bradykinin derivatives (e.g., Lys-BKm01, m02, m06, m12, m14 and other suitable PEG conjugates) with good retention of activity of bradykinin receptor affinity and activating downstream signaling pathways. They improved the impaired motor coordination function, forelimb muscle strength, working memory, spatial learning and memory function, and depressive behavior of BCAS model mice. In contrast, bradykinin derivatives (e.g., PEG conjugates of Lys-BKm05, m07, etc.), which lost much of the activity of the bradykinin receptor affinity and activating downstream signaling pathways, did not show similar effects.

In conclusion, bradykinin derivatives with good retention of activity of receptor affinity and activating downstream signaling pathway can be used for the treatment of vascular cognitive impairment, post-stroke cognitive impairment, and cerebral small vessel disease.

It should be noted that peptide drugs generally have poor stability and short half-life in vivo, and lysyl-bradykinin and bradykinin are more unstable than common peptides, with a half-life of only a few seconds, and exogenously administered kinin is rapidly inactivated by kallikrein hydrolysis in vivo and cannot be used as a drug. Therefore, only the long-acting lysyl bradykinin and bradykinin are pharmaceutically viable. Although the long-acting methods of the examples were PEG coupling, the long-acting methods of lysyl bradykinin and bradykinin were not the primary problem to be solved in the present application, and the PEG coupling technology used in the specific embodiments was only used for illustrative purposes but not to limit the long-acting methods of the present application. Those skilled in the art can expect that lysyl bradykinin/bradykinin derivatives with other long-acting techniques, good retention of activity of receptor affinity and activating downstream signaling pathway can improve the impaired function of BCAS model mice and be used to treat vascular cognitive impairment, post-stroke cognitive impairment, and cerebral small vessel disease.

Furthermore, examples illustrated the stability, in vitro activity, and in vivo efficacy of PEG modified bradykinin lysyl, bradykinin or its variants, all of which showed good stability. Moreover, after polyethylene glycol modification, lysyl bradykinin or bradykinin variants with retained activity in vitro can prolong the half-life of lysyl bradykinin or bradykinin and exerted its bioactivity in vivo. In contrast, other lysyl bradykinin or bradykinin variants that did not retain in vitro activity or PEG modified variants that did not retain in vitro activity were not active in vivo. Therefore, one skilled in the art can reasonably expect that other long-acting lysyl bradykinin or bradykinin derivatives that retain receptor binding activity can solve the problem of prolonging the half-life of lysyl bradykinin or bradykinin, exert its in vivo biological activity, and improve the impaired function of BCAS model mice, therefor can be used for the treatment of vascular cognitive impairment, post-stroke cognitive impairment, and cerebral small vessel disease, and not limited to the specific lysyl bradykinin or bradykinin variant of the example.

## Claims

1. The use of kinin or its derivatives in the preparation of drugs for the treatment or improvement of vascular cognitive impairment.

2. A pharmaceutical composition for the treatment or improvement of vascular cognitive impairment, comprising kinin or a derivative thereof.

3. A method of treating or improving vascular cognitive impairment, in which the patient is given kinin or a derivative thereof.

4. The use of kinin or its derivatives in the preparation of drugs for the treatment or improvement of post-stroke cognitive impairment.

5. A pharmaceutical composition for treating or improving post-stroke cognitive impairment, comprising kinin or a derivative thereof.

6. A method of treating or improving post-stroke cognitive impairment, in which a patient is given kinin or a derivative thereof.

7. The use of kinin or its derivatives in the preparation of drugs for the treatment or improvement of Alzheimer's disease with vascular lesions.

8. A pharmaceutical composition for the treatment or improvement of Alzheimer's disease with vascular lesions, comprising kinin or aderivative thereof.

9. A method of treating or improving Alzheimer's disease with vascular lesions, in which the patient is given kinin or a derivative thereof.

10. The use of kinin or its derivatives in the preparation of drugs for the treatment or improvement of cerebral small vessel disease.

11. A pharmaceutical composition for the treatment or improvement of cerebral small vessel disease, comprising kinin or a derivative thereof.

12. A method of treating or improving cerebral small vessel disease, in which kinin or its derivative is administered to the patient.

13. The kinin according to any one of claim 1 to 12 is bradykinin or lysyl bradykinin.

14. The kinin derivative according to any one of claim 1 to 12 is long-acting kinin.

15. The kinin derivative of claim 14 is polyethylene glycol modified kinin, and the PEG modifier used is SPA, SCM or SS modifier; the PEG modifier used is linear or branched PEG modifier.

16. The kinin derivative of claim 14 is mutant of kinin, wherein cysteine is introduced at any position in the lysyl bradykinin or bradykinin sequence, and/or any amino acid is inserted between the first and second amino acid residue at the N-terminus of lysyl bradykinin, and/or any amino acid is inserted before the first amino acid residue at the N-terminus of bradykinin; and/or consists of two or more lysyl bradykinin monomers in series, or consists of two or more bradykinin monomers in series, or consists of lysyl bradykinin monomers and bradykinin monomers in series; and/or Phe at position 6 of lysyl bradykinin or Phe at position 5 of bradykinin is mutated to other amino acid.

17. The kinin derivative of claim 14, which is fusion protein, and the kinin is fused to Fc fragment, or to human serum albumin.

18. The kinin derivative of claim 14, which is kinin conjugated with fatty acids or long-acting formulation of kinin.
